# EUROPEAN PATENT SPECIFICATION

(11) **EP 2 036 499 B1**
(45) Date of publication and mention of the grant of the patent: **09.09.2015**
(21) Application number: 08016140.9
(22) Date of filing: 12.09.2008
(51) Int. Cl.: A61B 8/00, G01R 31/317, G01R 31/3185, G06F 19/00

(54) **Medical device and test method for medical device**
Medizinische Vorrichtung und Testverfahren dafür
Dispositif médical et procédé de test pour dispositif médical

(30) Priority: 12.09.2007 JP 2007237048
(43) Date of publication of application: 18.03.2009
(73) Proprietor: Olympus Medical Systems Corp., Tokyo 151-0072 (JP)
(72) Inventor: Yoshimura, Takehiro, Shibuya-ku, Tokyo 151-0072 (JP)
(74) Representative: von Hellfeld, Axel

(56) References cited:
- US-A1- 2003 040 820
- US-A1- 2005 099 832
- US-A1- 2006 282 734
- US-A1- 2007 011 528
- US-B1- 7 117 352

## Description

### BACKGROUND OF THE INVENTION

### 1. Field of the Invention

The present invention relates to a medical device and a test method for a medical device.

### 2. Description of the Related Art

Recently, as medical devices have become sophisticated and complex, it is getting not easy, when a medical device has a malfunction, to inspect and identify the location of the malfunction.

Therefore, a technique has been proposed: performing maintenance work or the like of a medical device from a remote location using communication lines in order to prevent a malfunction in advance.

For example, Japanese Patent Application Laid-Open Publication No. 2001-327497 discloses a remote maintenance method for a medical image diagnostic device, in which change of system setting of a medical image diagnostic device is performed by a computer at a remote location, connected through a network.

In addition, Japanese Patent Application Laid-Open Publication No. 2005-058574 discloses an ultrasound diagnostic device in which a service person can reliably and readily perform maintenance work of the main body side of the ultrasound diagnostic device on the remote side without going to the main body side of the ultrasound diagnostic device.

In the case of an ordinary device which is not a medical device, when a malfunction of the device occurs, a repairer can perform close inspection after disassembling the device having a malfunction on the site to find out the location of the malfunction.

However, specified medical devices such as an ultrasound diagnostic device must not only be manufactured or sold without permission by relevant authorities according to the law (such as Pharmaceutical Law), but must not be disassembled except in a place of business having a license for repairing business, equipped with buildings and facilities specified by Regulations for Buildings and Facilities for Pharmacies and the like.

US 2003/0040820 describes an imaging system with a programmable detector framing node controlling generation of radiation and controlling radioscopic image detection. The framing node is programmable via a pair of JTAG loops that receive programming instructions from a host computer and from a pair of JTAG ports. A plurality of eeprom units are daisy-chained and accessible via a single JTAG port. A push-button switch may force an override of the reset circuit, and various zero ohm resistors are provided to take either of the tested circuits out of the JTAG loop in case there is a problem during debugging or firmware development. The host computer is able to access the JTAG ports directly over the computer communication bus for remote programming. Error checking is used to ensure that the devices have been programmed correctly, however this will not prevent a user from programming the wrong firmware into the eeprom units. This situation is mitigated using-software interlocks and through general precaution. Alternatively, the eeprom units may be replaced.

US 2007/0011528 describes a medical imaging system including a plurality of circuit boards configured to be tested using boundary scan test vectors. A controller is configured to access test profiles to perform boundary scan tests on the plurality of circuit boards based on the test profiles in order to test the connectivity of a set of boards and/or a system of interconnected boards (i.e., system level testing). A controller stores boundary scan vectors, boundary scan test profiles, and boundary scan test results, including procedures to perform various continuity and system level tests of the interconnected components of the ultrasound system. Individual boards may be tested at a manufacturing facility, however, the interconnecting set of boards is typically not or cannot be tested at the manufacturing facility. Boundary scan testing of the interconnected boards of the medical imaging system may be performed after the system is deployed, for exampled, at a customer site. The boundary scan test vectors may be supplied by the manufacturers of the components of the imaging system, which may be modified as desired or needed.

US 2006/0282734 describes a system for providing testing access to an integrated circuit controlled by the user of test access enabling keys, which may be performed be dedicated hardware or software executing a secure privilege mode. The testing method may also allow managed control of access to different levels of test access.

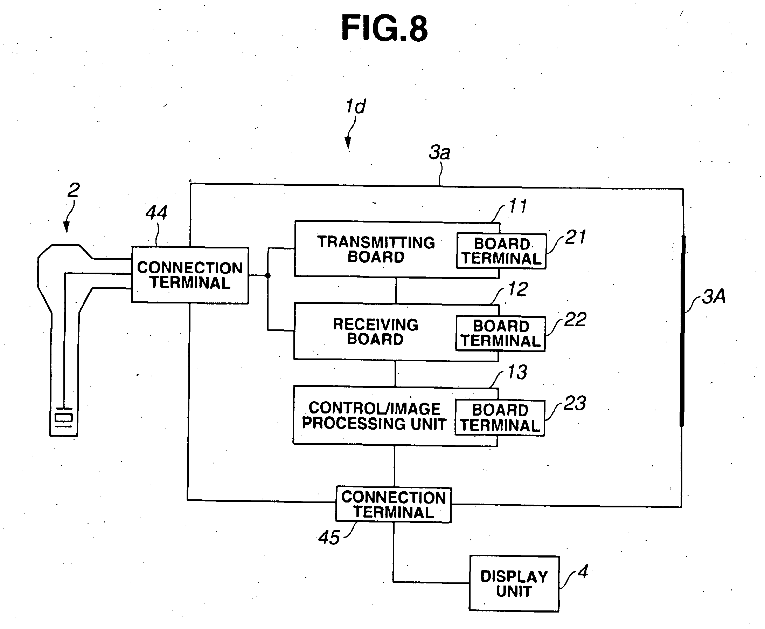

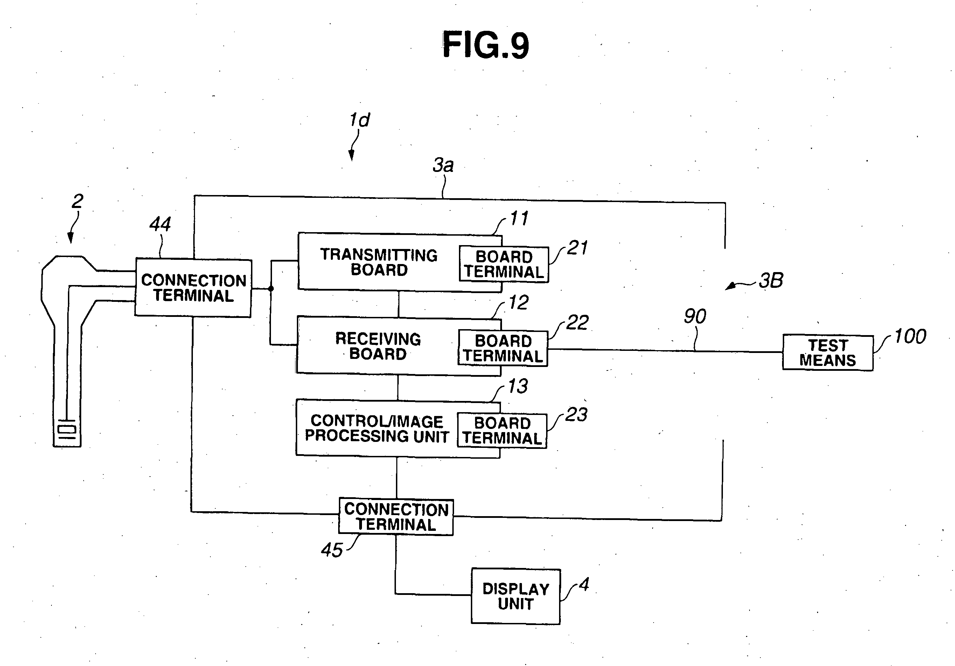

Fig. 8 is a diagram of the configuration of a conventional ultrasound diagnostic device 1d, and Fig. 9 is a diagram of the configuration at the time of testing the conventional ultrasound diagnostic device 1d.

As shown in Figs. 8 and 9, in order to test wiring boards 11, 12, 13 disposed in a housing 3a of the device of the ultrasound diagnostic device 1d, an outer surface panel 3A covering the housing 3a needs to be removed to connect board terminals 21, 22 or 23 and test means 100 through an opening portion 3B.

As described above, however, the outer surface panel 3A of the ultrasound diagnostic device 1d, which is a medical device, can not be removed on the site, that is, disassembly work can not be performed. Therefore, as an on-site test for discovering a malfunction, only a test by checking of operation or the like with operating buttons (not shown) or the like provided on the outer surface of the housing 3a can be performed. Or, what can be done is at most to determine whether the cause of the malfunction is in the ultrasound endoscope or in the wiring boards disposed in the housing 3a, by replacing an ultrasound endoscope 2, which is removable by means of a connection terminal 44 from the housing 3a, with another ultrasound endoscope.

Therefore, among the many wiring boards in a housing, identification of the wiring board having mounted a semiconductor component such as an LSI having an abnormality, that is, separation of the cause of a malfunction in a housing is impossible. Consequently, when a malfunction occurs in a medical device, the main body of the medical device needs to be brought to a place of business having a license for repairing business.

On the other hand, sophisticated medical devices such as an ultrasound endoscope are indispensable in medical fields; non-availability of such a medical device for a long time may cause trouble in medical examinations, treatments and the like.

In addition to the aspect of hardware as described above, a medical device having permission according to the law is not allowed to modify the software such as programs recorded on semiconductor components therein unless permitted by relevant authorities. Therefore, even if a modification of the software is an unintended one due to an operation error by a person performing a test, the modified medical device is deprived of permission and becomes unusable for medical practice.

Of course, also in the case of testing a medical device using communication lines from a remote location as described above, modification of the software of the medical device is sometimes not allowed since it violates ordinances. For this reason, a medical device free from the possibility of modification of the software has been desired.

### SUMMARY OF THE INVENTION

These problems are solved by a medical device and test method according to the independent claims.

An ultrasound diagnostic device which is a medical device of the present invention is a medical device having a plurality of wiring boards disposed in a housing of a device, each of which has a board terminal for testing the wiring board, comprising: one or more external terminals provided on an outer surface of the housing of the device and connected to one or more of the board terminals; and one or more block means provided between the board terminal and the external terminal for blocking writing of data to the wiring boards.

Additionally, a test method for an ultrasound diagnostic device which is a medical device of the present invention is a test method for a medical device having a plurality of wiring boards disposed in the housing of a device, each of which has a board terminal for testing the wiring board, comprising one or more external terminals provided on the outer surface of the housing of the device and connected to one or more of the board terminals, wherein: test means is connected to one or more external terminals; one or more block means for blocking writing of data to the wiring boards is disposed between the board terminal and the test means; and the test means performs testing of the wiring boards through the external terminal while writing of data to the wiring boards is blocked.

### BRIEF DESCRIPTION OF THE DRAWINGS

Fig. 1 is a diagram of a configuration of an ultrasound diagnostic device of a first embodiment of the present invention;
Fig. 2 is a schematic function block diagram of data writing block means of the first embodiment of the present invention;
Fig. 3 is a diagram showing an example of connection of a wiring board having a board terminal of the JTAG standard;
Fig. 4 is a flow chart for performing testing of a wiring board using the board terminal of the JTAG standard;
Fig. 5 is a diagram of a configuration for describing a test method for a medical device of the first embodiment;
Fig. 6 is a diagram of a configuration of a medical device of a second embodiment of the present invention;
Fig. 7 is a diagram of a configuration showing a test method for a medical device of a third embodiment of the present invention;
Fig. 8 is a diagram of a configuration of a conventional ultrasound diagnostic device; and
Fig. 9 is a diagram of a configuration at the time of performing testing of a conventional ultrasound diagnostic device.

### DETAILED DESCRIPTION OF THE PREFERRED EMBODIMENTS

Embodiments of the present invention will be described below with reference to the drawings.

### <First embodiment>

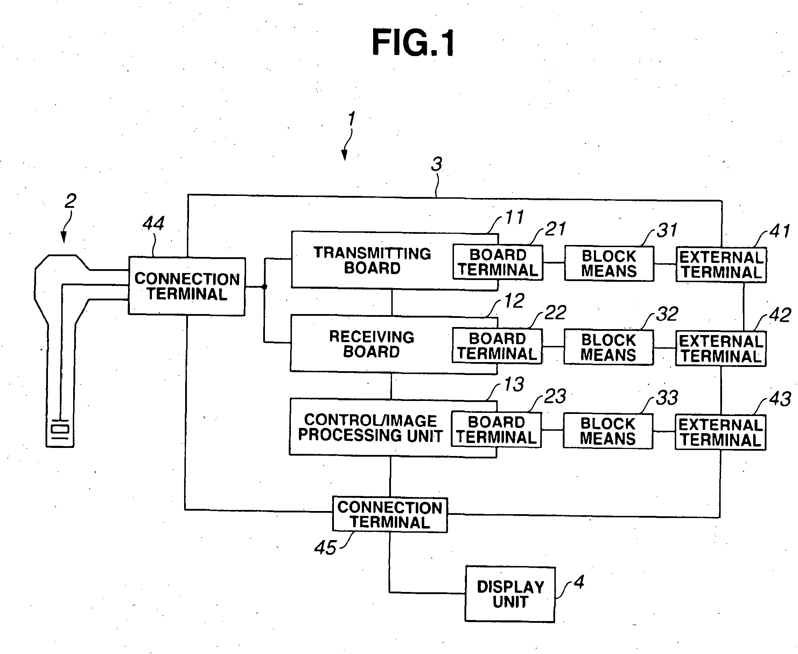

Fig. 1 is a diagram of a configuration of an ultrasound diagnostic device of a first embodiment of the present invention.

As shown in Fig. 1, an ultrasound diagnostic device 1 which is a medical device of the present embodiment has a housing of the device (hereinafter referred to as "housing") 3, an ultrasound endoscope 2 and a display unit 4. The housing 3 has a connection terminal 44 to which the ultrasound endoscope 2 is attachable and detachable and a connection terminal 45 to which the display unit 4 is attachable and detachable. In addition, the housing 3 has three external terminals 41, 42, 43 on the outer surface of the housing.

The housing 3 is a box covered by outer walls, accommodating control means and the like of the ultrasound diagnostic device: a box which must not be disassembled except in a place of business having a license for repairing business, equipped with buildings and facilities specified by Regulations for Buildings and Facilities for Pharmacies and the like according to the law (such as Pharmaceutical Law), as described above. That is, the housing 3 refers to members constituting the outer walls, except members removal of which causes no problem from a legal standpoint such as a readily removable cover for preventing contamination and a readily removable panel for preventing erroneous operation.

In the housing 3, a plurality of wiring boards are disposed on which various semiconductor components and the like are mounted. As examples of major wiring boards, Fig. 1 shows three wiring boards: a wiring board 11 for transmitting processing, a wiring board 12 for receiving processing and a wiring board 13 for control/image processing. Each of the above wiring boards has FPGA (Field Programmable Gate Array) function and provides each function by programming.

The wiring boards 11, 12, 13 of the ultrasound diagnostic device 1, which is a medical device of the present embodiment, are provided with board terminals 21, 22, 23 for testing the various semiconductor components mounted on the wiring boards, respectively. These board terminals are board terminals which have come to be provided since it has become difficult to connect a test probe to each of the terminals to perform testing because the semiconductor components have come to have many terminals in order to adapt to high-density mounting; and are hereinafter referred to as board terminals.

That is, in Fig. 1, each of the wiring boards 11, 12, 13 has a board terminal and each board terminal is connected to an external terminal. Specifically, the board terminals 21, 22, 23 are respectively connected to the external terminals 41, 42, 43. However, not all the wiring boards in the housing 3 need to have a board terminal. In addition, there may be a wiring board having a board terminal but being not connected to an external terminal. That is, only board terminals which need to be tested without disassembling the housing 3 may particularly be connected to external terminals, considering the frequency of occurrence of malfunctions of each wiring board or the like.

To be exact, as shown in Fig. 1, the external terminals 41, 42, 43 are provided between the inner surface and the outer surface of the housing 3, and are connected to the board terminals 21, 22, 23 through block means 31, 32, 33 in the inner surface of the housing 3.

Each of the three board terminals in the housing 3 of the present embodiment is connected to an external terminal through the block means. The data writing block means 31, 32, 33 are provided between the board terminals 21, 22, 23 on the three wiring boards and the external terminals 41, 42, 43 on the outer surface of the housing 3, corresponding to the respective board terminals and the respective external terminals.

In the following, each of the board terminals 21, 22, 23 is referred to as a board terminal 20, each of the block means 31, 32, 33 is referred to as block means 30, and each of the external terminals 41, 42, 43 is referred to as an external terminal 40. Each of the board terminals 20 and each of the external terminals 40 represent a terminal group respectively having a plurality of physical terminals for connecting a plurality of wires. A board terminal of the JTAG standard described later is, for example, a terminal group comprising four wires and one option wire: a total of five wire terminals.

The block means 30 is provided for each board terminal 20 because the board terminal 20 for testing the wiring board not only can test the wiring board, that is, test the semiconductor components (such as CPU, FPGA and EPROM) mounted on the wiring board, but can also rewrite or newly write the data such as programs and parameters recorded in the semiconductor components to be tested, through the board terminal 20.

In the case of an ordinary device other than medical devices, function such as rewriting of data is useful function for easily performing version upgrade work of the software. However, such function of the board terminal causes a problem in testing the wiring boards of a medical device as described above.

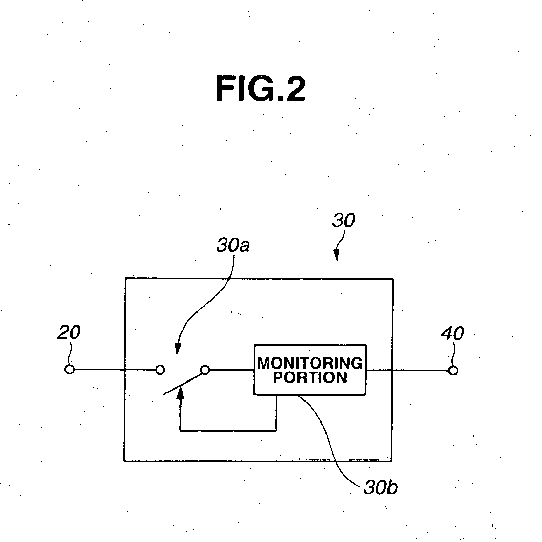

Fig. 2 shows a schematic function block diagram of the data writing block means 30 of the present embodiment. The block means 30 is provided between the board terminal 20 and the external terminal 40. The block means 30 comprises a switch portion 30a and a monitoring portion 30b. The monitoring portion 30b always monitors whether or not there is a signal to write data to the semiconductor components mounted on the wiring board. Operation of writing data to the semiconductor components on the wiring board is, from the standpoint of the semiconductor components, reading of data.

One end of the monitoring portion 30b is connected to the external terminal 40 and the other end is connected to the board terminal 20 through the switch portion 30a. The switch portion 30a is switched ON or OFF depending on a scan signal from the monitoring portion 30b. For example, the switch portion 30a is usually ON: the signal from the external terminal 40 being supplied to the board terminal 20 and also the signal from the board terminal 20 being supplied to the external terminal 40. But when the signal from the external terminal 40 is a signal to write data to a memory of a semiconductor component in the wiring board, the monitoring portion 30b detects that writing signal and outputs a control signal for the switch portion 30a to be OFF.

Specifically, the block means 30 always monitors a signal to start writing of data to the wiring board, and stores in a predetermined memory area in the block means 30 flag data in which the state of no signal to start writing of data is 0, whereas the state of detecting a signal to start writing of data is 1. The block means 30 switches the switch portion 30a ON, that is, providing connection between the external terminal 40 and the board terminal 20 when the flag is 0, but switches the switch portion 30a OFF, that is, providing release between the external terminal 40 and the board terminal 20 to block writing of data when the flag is 1.

Here, the signal to start writing of data to the wiring board is a signal transmitted at the beginning of the start of operation of writing data from the outside to a memory in a semiconductor component mounted on the wiring board. The type of data of the signal to start writing of data differs depending on the kinds of the semiconductor components, the dedicated applications for testing the wiring board or the like. Therefore, the block means detects the signal to start writing of data corresponding to the medical device to be tested.

From the viewpoint of the fail-safe at the time of a malfunction of the block means, preferably the block means 30 usually keeps the switch portion 30a OFF, and provides connection only when the monitoring portion 30b can confirm that there is no signal to start writing.

The ultrasound diagnostic device 1 which is a medical device of the present embodiment has the block means between the board terminal and the external terminal. Therefore, testing of the semiconductor components mounted on the wiring board can be appropriately performed using the board terminal in a medical device whose modification of the software is not permitted.

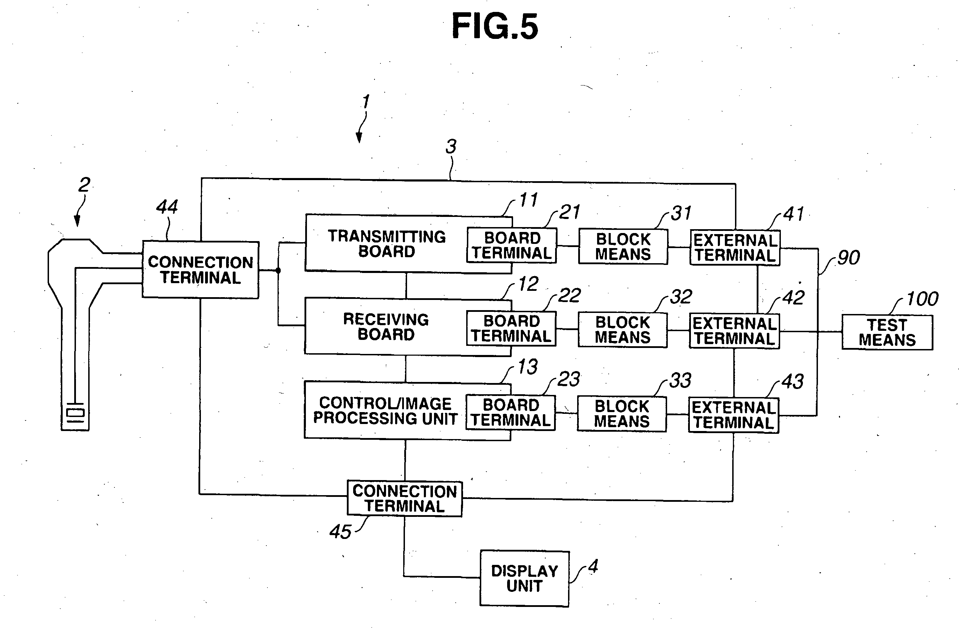

For the board terminals 21, 22, 23, the check board terminal of the JTAG standard is preferable. In the following, an example of a medical device will be shown in which the check board terminal of the JTAG standard is used as the board terminal for testing the wiring board. Fig. 5 is a diagram of a configuration for describing a test method for the ultrasound diagnostic device 1 which is a medical device of the first embodiment.

The JTAG standard was proposed by Joint European Test Action Group (JETAG: now JTAG) in 1985, and was established as a standard by Institute of Electrical and Electronics Engineers (IEEE) in 1990 as IEEE std. 1149.1-1990 "Standard Test Access Port and Boundary-Scan Architecture", setting forth a standard of the "boundary scan test" (boundary scan examination). The JTAG standard is a standard established for the purpose of solving problems arising from the difficulty in connecting probes physically to semiconductor components on which the BGA (Ball Grid Array) is mounted or to multilayer printed boards having many input/output pins, and performing testing.

A serial bus of the JTAG standard provides means for access from the test means for performing a boundary scan on each of the semiconductor components mounted on a wiring board. Therefore, the test means can test the inactive semiconductor components. By the test, the test means can verify the connection of the semiconductor components and can verify that they are properly attached and interconnected, that is, can test the wiring board.

A serial bus of the JTAG standard can interconnect one or more semiconductor components in the form of a chain, and the test means can designate any of the semiconductor components by addresses. Since usually a plurality of devices of the wiring board are interconnected to the JTAG bus, the serial bus of the JTAG standard is sometimes called a JTAG chain.

The serial bus of the JTAG standard uses four lines and one option line. These lines include a serial data input line, a serial data output line, a clock line and a test mode select line. Usually, the data output line of the first semiconductor component of the chain is connected to the data input line of the second semiconductor component of the chain, and the data output line of the second semiconductor component is connected to the data input line of the third semiconductor component. That is, the data input lines and the data output lines of a plurality of semiconductor components are connected in a daisy chain configuration.

IEEE1152 is an extended new version of 1149.1JTAG. In the present invention, the JTAG includes both variations of 1149.1 and 1152, and also includes versions having further developed similar function.

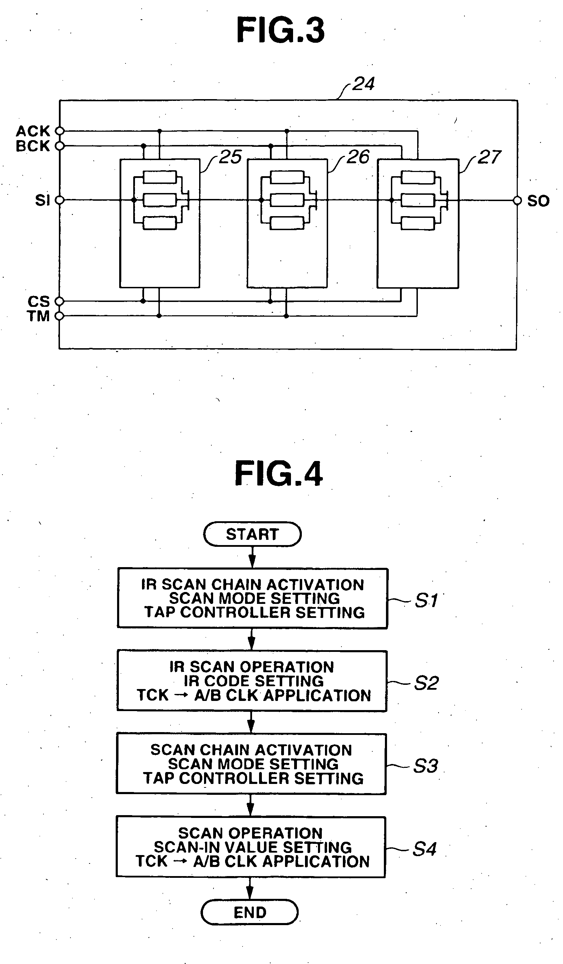

Fig. 3 is a diagram showing an example of connection of a wiring board 24 having a board terminal of the JTAG standard. In Fig. 3, LSI 25, 26 and 27, which are semiconductor components mounted on the wiring board 24, have provided therein control signal generating means including an unshown instruction register and scan A/B clock generating means which generates clocks of two phases of scan A/B phases. The state of the control means in the semiconductor components LSI 25, 26 and 27 is set by a sequence signal of the connected test means to perform a scan.

In Fig. 3, ACK is a terminal for the A clock signal and BCK is a terminal for the B clock signal. A scan board terminal SI is a terminal for inputting a scan signal and a scan output terminal SO is a terminal for outputting the scan signal. A chain select CS is a chain select terminal for selecting a range connected in the same scan chain, and a test mode select signal board terminal TM is a terminal for a signal which selects a test mode. The above terminal group corresponds to the board terminal 20.

Fig. 4 shows a flow chart for testing the wiring board 24 using the board terminal of the JTAG standard. Specifically, in the flow chart shown in Fig. 4, in order to activate the chain connection of the instruction register scan system in which instruction registers (referred to as IR register) for setting instructions for performing testing are serially connected, first the scan mode is set and a TAP controller, which is JTAG test system control means, is set (S1). After the scan mode has been set and the TAP controller has been set, in order to perform scan operation of the instruction register, instruction register code is set and input voltage is applied from the test clock terminal to the scan A/B phase clock terminal (S2).

After the setting of the instruction register code and the voltage application from the test clock terminal to the scan A/B clock terminal, in order to activate the chain connection of the scan, the scan mode is set and the TAP controller is set (S3). After the scan mode has been set and the TAP controller has been set, in order to perform scan operation, a scan-in value to be inputted to the scan board terminal is set and input voltage is applied from the test clock terminal to the scan A/B clock terminal (S4). Thus a signal for setting the scan-in value to be inputted to the scan board terminal is provided and input voltage is applied from the test clock terminal to the scan A/B clock terminal, ending the test processing. The above test is automatically performed in accordance with content set in advance upon activation of a JTAG application which is application software for the JTAG test and which the test means 100 has.

As shown in Fig. 5, the three wiring boards 11, 12, 13 disposed in the housing 3 of the ultrasound diagnostic device 1 respectively have the board terminals 21, 22, 23 of the JTAG standard. The board terminals 21, 22, 23 of the JTAG standard on the wiring boards 11, 12, 13 are respectively connected through the block means 31, 32, 33 to the external terminals 41, 42, 43 provided on the outer surface of the housing 3. Here, the outer surface of the housing 3 of the device is the outer wall surface of the device.

First, the test means 100 equipped with the JTAG application is connected to the external terminals 41, 42, 43 through a cable 90. Although the test means 100 is connected to the three external terminals 41, 42, 43 in Fig. 5, it may be connected only to one or two external terminals to which the board terminal of the wiring board to be tested is connected.

Since the ultrasound diagnostic device 1 which is a medical device of the present embodiment is a medical device, the housing of the device cannot be disassembled on the site. However, since the outer surface of the housing 3 has the external terminals, the test means 100 can be connected to the board terminal of each wiring board through the external terminal.

Specifically, in the medical device of the present embodiment, the test means 100 equipped with a JTAG interface and a check application, for example a PC, can be connected to the external terminal 40 for the JTAG check on the outer surface of the housing 3. When a general-purpose PC not containing a JTAG interface is used as the test means 100, connection is made with a JTAG interface being interposed between the external terminal 40 for the JTAG check and the PC.

Next, the JTAG application mounted on the test means 100 is activated, an initial setting of test content adapted to the medical device to be tested is made and an actual test is performed.

Therefore, condition of the wiring boards in the device can be easily tested from the outside to diagnose a malfunction without disassembling the housing 3 on the site. In addition, when it is found that the cause of the malfunction exists in a particular wiring board accommodated in the housing 3, the main body of the device accommodating that wiring board is taken to a factory permitted to repair medical devices to perform disassembly and exchange work. When the device is taken to the factory, the location of the malfunction has been found in advance so that the time for disassembly work can be minimized, thereby improving the electrical safety of repair work. In addition, reduction of cost for maintenance and tests of the medical device becomes possible.

Further, since the wiring board having a malfunction is found, if that malfunctioning part is not a part of essential function of the medical device, often other function can be used without problems as long as the function in question is not used. For example, if it is found that the wiring board for controlling color Doppler function is the cause of the malfunction in an ultrasound diagnostic device, it is found that normal endoscope function and B-mode ultrasound diagnosis can be used safely. Therefore it is possible to get out of the worst situation of the medical device being wholly unusable.

In the ultrasound diagnostic device 1 which is a medical device of the present embodiment, the test means 100 is connected to the board terminal 20 of the JTAG standard without disassembling the ultrasound diagnostic device 1 to perform a test so that whether or not the wiring board has a malfunction can be tested appropriately and quickly without risk of modification of data.

### <Second embodiment>

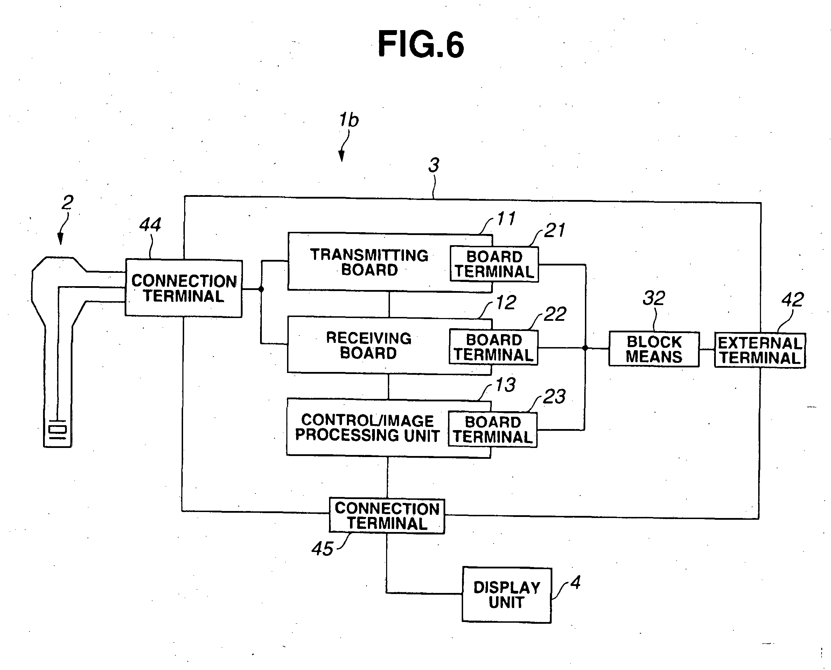

Fig. 6 is a diagram of the configuration of a medical device 1b of a second embodiment of the present invention.

The difference from the first embodiment is that three board terminals 21, 22, 23 on three wiring boards are connected to one block means 32 which is then connected to one external terminal 42 on the outer surface of a housing 3.

A complicated medical device uses a large number of wiring boards and therefore has a large number of the external terminals 40 provided on the outer surface of the housing 3. In addition, if the block means 30 is provided for each of the board terminals 20 as shown in the first embodiment, a large number of block means 30 are needed.

For this reason, in the medical device of the second embodiment, the board terminals 21, 22, 23 for the JTAG check on the wiring boards are connected to one block means 32, and the one block means 32 is connected to one external terminal 42 to achieve the same object as the first embodiment.

### <Third embodiment>

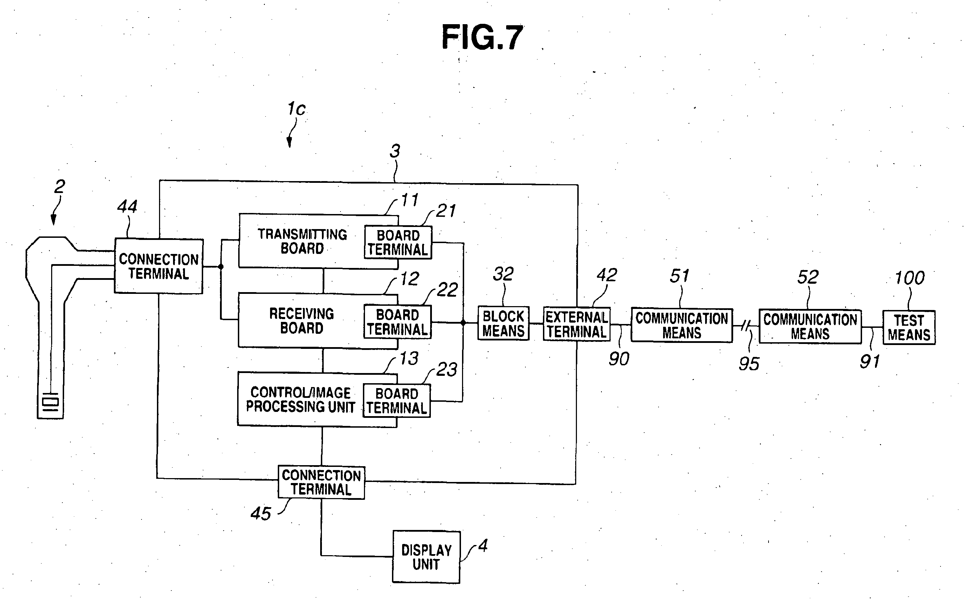

Fig. 7 is a diagram of a configuration showing a test method for a medical device 1c of a third embodiment of the present invention. In the present embodiment, communication means 51 having communication function is connected to an external terminal 42 on the outer surface of a housing 3, and is connected through a communication line 95 via communication means 52 to the test means 100.

By connecting the test means 100 to the external terminal 42 through the communication means 51 and 52, the medical device 1c can be tested from a remote location. For the communication line 95, LAN, the Internet or a cellular phone line is preferably used.

In the present embodiment, the block means 32 only needs to be disposed between the board terminal 20 and the test means 100. That is, the block means 32 does not need to be disposed in the housing 3.

The test method for the medical device of the present embodiment is a test method for a medical device having: a plurality of wiring boards disposed in the housing of the device, each of the wiring boards having a board terminal for testing the wiring board; and one or more external terminals provided on the outer surface of the housing of the device and connected to one or more of the board terminals, wherein: one or more block means for blocking writing of data to the wiring board is disposed between the board terminal and the test means; the test means is connected to one or more external terminals; and data is read from the wiring board by the test means through each external terminal while writing of data to the wiring board is blocked.

By the test method for the medical device of the present embodiment, a test on the medical device is performed via a communication line, and thereby further cost for maintenance and tests can be reduced as well as a business model for a new service can be built.

Information obtained by operation of medical devices, such as ultrasound diagnostic images, is personal information of a patient and must be strictly managed. Therefore, in the medical device of the present embodiment, a control portion for operational function of the medical device and a portion for control and storage of information obtained by operation of the medical device are preferably independent separate components.

The present invention has been described thus far assuming the current legal system. However, a situation is expected to occur where partial version upgrade work of software of medical devices on site is possible due to an amendment of the law in the future. In such case, data can be written to the semiconductor components and the like mounted on the wiring board temporarily by stopping the function of the block means 30 of the medical device, or by disposing means to be connected to the test means 100 not via the block means 30 is provided and switching the connected means.

Having described the preferred embodiments of the invention referring to the accompanying drawings, it should be understood that the present invention is not limited to those precise embodiments and various changes and modification thereof could be made by one skilled in the art without departing from the scope of the invention as defined in the appended claims.

## Claims

1. A medical device (1) having a plurality of wiring boards (11, 12, 13) disposed in a housing (3) of the device (1), each of which has a board terminal (20) for testing the wiring board (11, 12, 13), including:
one or more external terminals (40) provided on an outer surface of the housing (3) of the device (1) and connected to one or more of the board terminals (20);
**characterized by** comprising
one or more block means (30) provided between the board terminal (20) and the external terminal (40) and blocking writing of data to the wiring board (11, 12, 13).

2. The medical device according to claim 1, wherein the board terminal (20) for testing the wiring board (11, 12, 13) is a board terminal (20) of JTAG standard.

3. The medical device according to claim 1 or 2, wherein the number of the external terminals (40) is one.

4. The medical device according to claim 1, wherein the external terminal (40) is adapted to be connected to communication means (51, 52).

5. The medical device according to claim 1 or 4, wherein the medical device (1) is an ultrasound diagnostic device.

6. A test method for a medical device having: a plurality of wiring boards (11, 12, 13) disposed in a housing (3) of a device (1), each of which has a board terminal (20) for testing the wiring board (11, 12, 13) the testing method comprising:
a test means connecting step of connecting test means (100) to one or more external terminals provided on an outer surface of the housing (3) of the device (1) and connected to one or more of the board terminals (20);
a test step of testing the wiring board (11, 12, 13) by use of the test means (100) through the external terminal (40) while blocking writing of data to the wiring board (11, 12, 13) by use of one or more block means (30) disposed between the board terminal (20) and the test means (100) and blocking writing of data to the wiring board (11, 12, 13).

7. The test method for a medical device (1) according to claim 6, wherein the board terminal (20) for testing the wiring board (11, 12, 13) is a board terminal of JTAG standard.

8. The test method for a medical device (1) according to claim 6 or 7, further having:
a communication means (51, 52) connecting step of connecting communication means (51, 52) to the external terminal (40); and
a communication test means connecting step of connecting the test means (100) to the external terminal (40) through a communication line (95).

## Patentansprüche

1. Medizinische Vorrichtung (1) mit einer Vielzahl von Leiterplatten (11, 12, 13), die in einem Gehäuse (3) der Vorrichtung (1) angeordnet sind, wobei jede der Vielzahl von Leiterplatten einen Leiterplattenanschluss (20) zum Testen der Leiterplatte (11, 12, 13) aufweist, wobei die Vorrichtung (1) umfasst:
einen oder mehrere Außenanschlüsse (40), die auf einer Außenoberfläche des Gehäuses (3) der Vorrichtung (1) vorgesehen sind und mit einem oder mehreren der Leiterplattenanschlüsse (20) verbunden sind;
**dadurch gekennzeichnet, dass**
die Vorrichtung ferner ein oder mehrere Blockiermittel (30) umfasst, die zwischen dem Leiterplattenanschluss (20) und dem Außenanschluss (40) vorgesehen sind und ein Schreiben von Daten auf die Leiterplatte (11, 12, 13) blockieren.

2. Medizinische Vorrichtung nach Anspruch 1, wobei der Leiterplattenanschluss (20) zum Testen der Leiterplatte (11, 12, 13) ein Leiterplattenanschluss (20) des JTAG-Standards ist.

3. Medizinische Vorrichtung nach Anspruch 1 oder 2, wobei die Anzahl der Außenanschlüsse (40) eins beträgt.

4. Medizinische Vorrichtung nach Anspruch 1, wobei der Außenanschluss (40) dazu eingerichtet ist, mit Kommunikationsmitteln (51, 52) verbunden zu sein.

5. Medizinische Vorrichtung nach Anspruch 1 oder 4, wobei die medizinische Vorrichtung (1) ein Ultraschall-Diagnosegerät ist.

6. Testverfahren für eine medizinische Vorrichtung mit: einer Vielzahl von Leiterplatten (11, 12, 13), die in einem Gehäuse (3) der Vorrichtung (1) angeordnet sind, wobei jede der Vielzahl von Leiterplatten einen Leiterplattenanschluss (20) zum Testen der Leiterplatte (11, 12, 13) aufweist, wobei das Testverfahren umfasst:
einen Testmittelverbindungsschritt zum Verbinden von Testmitteln (100) mit einem oder mehreren Außenanschlüssen, die auf einer Außenoberfläche des Gehäuses (3) der Vorrichtung (1) vorgesehen sind und mit einem oder mehreren der Leiterplattenanschlüsse (20) verbunden sind;
einen Testschritt zum Testen der Leiterplatte (11, 12, 13) über den Außenanschluss (40) durch die Testmittel (100), während ein Schreiben von Daten auf die Leiterplatte (11, 12, 13) durch ein oder mehrere Blockiermittel (30) blockiert wird, die zwischen dem Leiterplattenanschluss (20) und den Testmitteln (100) angeordnet sind, und Blockieren des Schreibens von Daten auf die Leiterplatte (11, 12, 13).

7. Testverfahren für eine medizinische Vorrichtung (1) nach Anspruch 6, wobei der Leiterplattenanschluss (20) zum Testen der Leiterplatte (11, 12, 13) ein Leiterplattenanschluss des JTAG-Standards ist.

8. Testverfahren für eine medizinische Vorrichtung (1) nach Anspruch 6 oder 7, das ferner umfasst:
einen Kommunikationsmittelverbindungsschritt zum Verbinden von Kommunikationsmitteln (51, 52) mit dem Außenanschluss (40); und
einen Kommunikationstestmittelverbindungsschritt zum Verbinden der Testmittel (100) mit dem Außenanschluss (40) über eine Kommunikationsleitung (95).

## Revendications

1. Dispositif (1) médical ayant une pluralité de plaquettes de câblage (11, 12, 13) disposées dans un logement (3) du dispositif (1), chacune desquelles a une borne (20) de plaquette pour tester la plaquette de câblage (11, 12, 13), incluant :
une ou plusieurs borne(s) (40) externe(s) prévue(s) sur une surface extérieure du logement (3) du dispositif (1) et connectée(s) à une ou plusieurs des bornes (20) de plaquette ;
**caractérisé en ce que** comprenant
un ou plusieurs moyen(s) (30) de blocage prévu(s) entre la borne (20) de plaquette et la borne (40) externe et bloquant l'écriture de données sur la plaquette de câblage (11, 12, 13).

2. Dispositif médical selon la revendication 1, dans lequel la borne (20) de plaquette pour tester la plaquette de câblage (11, 12, 13) est une borne (20) de plaquette à la norme JTAG.

3. Dispositif médical selon la revendication 1 ou 2, dans lequel le nombre des bornes (40) externes est un.

4. Dispositif médical selon la revendication 1, dans lequel la borne (40) externe est adaptée à être connectée à des moyens (51, 52) de communication.

5. Dispositif médical selon la revendication 1 ou 4, dans lequel le dispositif (1) médical est un dispositif de diagnostic par ultrasons.

6. Procédé de test pour un dispositif médical ayant : une pluralité de plaquettes de câblage (11, 12, 13) disposées dans un logement (3) d'un dispositif (1), chacune desquelles a une borne (20) de plaquette pour tester la plaquette de câblage (11, 12, 13), le procédé de test comprenant :
une étape de connexion de moyen de test pour connecter un moyen (100) de test à une ou plusieurs borne(s) externe(s) prévue(s) sur une surface extérieure du logement (3) du dispositif (1) et connectée(s) à une ou plusieurs des bornes (20) de plaquette ;
une étape de test pour tester la plaquette de câblage (11, 12, 13) en utilisant le moyen (100) de test par l'intermédiaire de la borne (40) externe tout en bloquant l'écriture de données sur la plaquette de câblage (11, 12, 13) en utilisant un ou plusieurs moyen(s) (30) de blocage disposé(s) entre la borne (20) de plaquette et le moyen (100) de test et bloquant l'écriture de données sur la plaquette de câblage (11, 12, 13).

7. Procédé de test pour un dispositif (1) médical selon la revendication 6, dans lequel la borne (20) de plaquette pour tester la plaquette de câblage (11, 12, 13) est une borne de plaquette à la norme JTAG.

8. Procédé de test pour un dispositif (1) médical selon la revendication 6 ou 7, ayant en outre :
une étape de connexion de moyens (51, 52) de communication pour connecter des moyens (51, 52) de communication à la borne (40) externe ; et
une étape de connexion de moyen de test de communication pour connecter le moyen (100) de test à la borne (40) externe par l'intermédiaire d'une ligne (95) de communication.
